# EUROPEAN PATENT SPECIFICATION

(11) **EP 2 879 611 B1**
(45) Date of publication and mention of the grant of the patent: **08.01.2020**
(21) Application number: 13825603.7
(22) Date of filing: 30.07.2013
(51) Int. Cl.: A61B 17/56, A61G 13/10, G01B 5/008, A61B 34/20, A61B 17/17, A61B 90/00

(54) **METHOD AND SYSTEM FOR CREATING FRAME OF REFERENCE FOR CAS WITH INERTIAL SENSORS**
VERFAHREN UND SYSTEM ZUR ERZEUGUNG EINES REFERENZRAHMENS FÜR CAS MIT TRÄGHEITSSENSOREN
PROCÉDÉ ET SYSTÈME DE CRÉATION D'UN CADRE DE RÉFÉRENCE POUR CAO AVEC CAPTEURS INERTIELS

(30) Priority: 30.07.2012 US 201261677106 P
(43) Date of publication of application: 10.06.2015
(73) Proprietor: Orthosoft ULC, Montréal QC H3C 2N6 (CA)
(72) Inventor: PARADIS, François, Boucherville, Québec J4B 7V1 (CA); ZUHARS, Joel, Livermore, California 94550 (US); DUVAL, Karine, Montréal, Québec H2K 1E7 (CA); CHEVRIER, Mathieu, Roxboro, Québec H8Y 1M1 (CA); AMIOT, Louis-Philippe, Hampstead, Québec H3X 3G8 (CA); JANSEN, Herbert, Montréal, Québec H2H 2A6 (CA); VALIN, Myriam, Laval, Québec H7N 3M7 (CA); DYE, Don, Warsaw, Indiana 46581 (US); ROBITAILLE, Isabelle, Montréal, Québec H3C 2N6 (CA); FERRON-FORGET, Simon, Montréal, Québec H3C 2N6 (CA); BAUDOIN, François, Québec H3C 2N6 (CA)
(74) Representative: Taor, Simon Edward William
(86) International application number: PCT/CA2013/050590
(87) International publication number: WO 2014/019087

(56) References cited:
- WO-A1-2010/011978
- WO-A1-2012/027815
- US-A1- 2006 095 047
- US-A1- 2008 077 004
- US-A1- 2009 248 044
- US-A1- 2010 174 287
- US-A1- 2012 022 406
- US-A1- 2012 029 389
- US-A1- 2012 053 594

## Description

### FIELD OF THE APPLICATION

The present application relates to computer-assisted surgery using inertial sensors, for instance orthopedic surgery.

### BACKGROUND OF THE ART

Inertial sensors (e.g., accelerometers, gyroscopes, inclinometers, etc) are increasingly used in computer-assisted surgery for numerous reasons. Off-the-shelf inertial sensors are relatively inexpensive and produce results of sufficient precision and accuracy for orthopedic surgery applications.

A common trait of inertial sensors is that they often do not provide positional information but, rather, simply orientational information, as they operate relative to gravity. Therefore, methods must be devised to create bone references and tools considering the absence of positional information.

United States patent application US 2012/0022406 discloses a method and system for determining the orientation of a surgical tool with respect to a bone of a patient, and a device for positioning a sensor.

WO2010011978 discloses a landmark acquisition assembly comprising an orthopedic fixture which can be used to identify the location of an axial line or plane. The landmark acquisition assembly can comprise a structure or structures for contacting an anatomical landmark or landmarks in order to obtain an alignment of an axis or plane extending through those anatomical landmarks.

### SUMMARY OF THE APPLICATION

It is therefore an aim of the present invention to provide a novel method and system for creating a frame of reference for bones in computer-assisted surgery with inertial sensors.

According to the invention, there is provided a digitizer device as set out in claim 1.

According to an aspect of the invention, there is provided an assembly of a digitizer device and table reference device as set out in claim 10.

According to another aspect of the invention, there is a method as set out in claim 14.

Optional features are set out in the dependent claims.

### BRIEF DESCRIPTION OF THE DRAWINGS

Fig. 1 is a perspective view of a caliper instrument of a bone digitizer of the present disclosure;
Fig. 2 is a block diagram of the pelvic digitizer as part of a bone digitizing system of the present disclosure;
Fig. 3 is a flowchart of a method for creating a pelvic frame of reference with inertial sensors for subsequent tool navigation; and
Figs. 4A-4C are perspective views of a table reference locator in accordance with an embodiment of the present disclosure.

### DESCRIPTION OF THE EXEMPLARY EMBODIMENTS

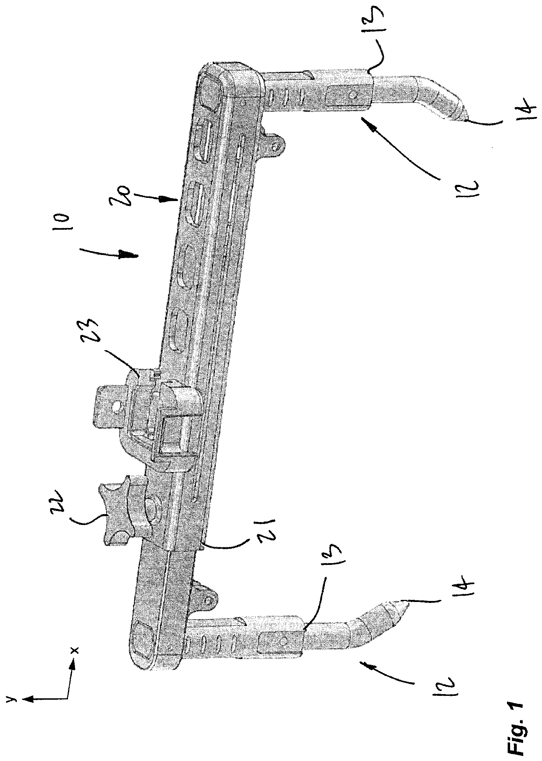

Referring to the drawings, and more particularly to Fig. 1, there is illustrated a caliper instrument 10 in accordance with the present application. The caliper instrument 10 may be used as part of a bone digitizer in a bone digitizing system, to create a frame of reference for subsequent navigation of tools relative to bones in surgery. The instrument 10 is referred to as a caliper, as it features a pair of legs 12 movable relative to one another, e.g., in a telescopic manner. The expression *"caliper"* is used nonrestrictively. Any other appropriate expression may be used to describe the instrument 10.
In the illustrated embodiment, the legs 12 of Fig. 1 each comprise a translational joint 13 so as to be expandable or contractible along the Y axis. For instance, the translational joints 13 may be any of sliding joint, telescopic joint, prismatic joint, indexing joint, etc. As an alternative, a single one of the legs may have a joint. It is also considered to use rotational joints as an alternative to translational joints 13, with an axis of the rotational joint being normal to a plane of the caliper instrument 10. A locking mechanism is typically provided, although not shown, to lock the translational joints 13 and, therefore, set the legs 12 in a selected length. The free end of each leg 12 has a pointy shape 14, although any other appropriate shape is considered, such as flat contact surfaces, discs, various concavities or convexities, etc., as a function of the type of bone or bodily part the caliper instrument 10 will be contacting. The pointy ends 14 of Fig. 1 are well suited to be used with a pelvis, with the pointy ends 14 contacting the anterior superior iliac spines (ASIS) on opposite sides of the pelvis, in pelvic surgery, with the patient in supine decubitus. Alternatively, the caliper instrument 10 could be used for the posterior superior iliac spine as well, or with other landmarks if the patient is in lateral decubitus.

Still referring to Fig. 1, the legs 12 are interconnected by an elongated body 20 of the caliper instrument 10. The elongated body 20 features a translational joint 21 such that the elongated body 20 is expandable or contractible along the X axis. The translational joint 21 may be any appropriate joint, such as translational joints, telescopic joint, prismatic joints and/or indexing joints. It is also considered to use rotational joints as an alternative to the translational joint 21.

A locking device is generally shown at 22, and is of the type having a manual knob used to set the translational joint 21 in at a selected length, thereby allowing the user to set the length of the elongated body 20. An inertial sensor support or receptacle 23 is defined on the elongated body 20. The inertial sensor support 23 is, for instance, made with a specific geometry in order to precisely and accurately accommodate an inertial sensor unit in a predetermined complementary connection, simplifying a calibration between inertial sensor unit and caliper instrument 10. For instance, the inertial sensor unit has a preset orientation that is aligned with a dimension of the caliper instrument 10. In other words, the mechanical constraints in the attachment of inertial sensor unit 31 in the support 23 are such that the three axes of the inertial sensor unit 31 are aligned with the X, Y and Z axis of the caliper instrument 10. Therefore, the caliper instrument illustrated in Fig. 1 may expand and contract along both the X axis and the Y axis.

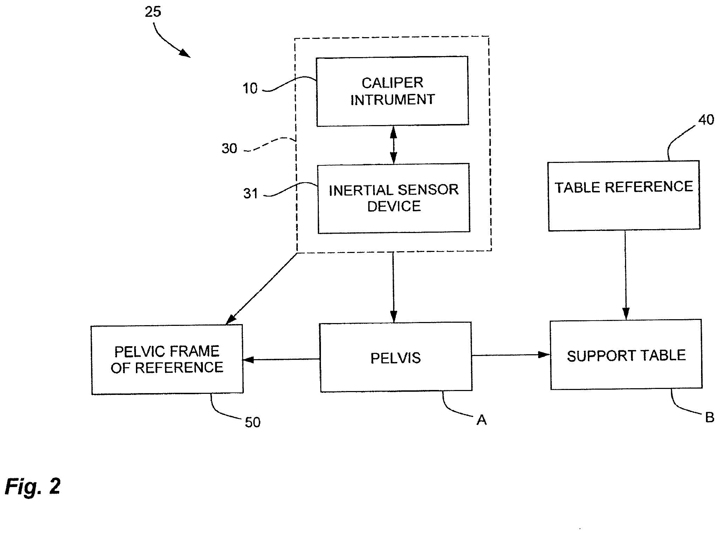

Referring to Fig. 2, the caliper instrument 10 is used as an instrument of a bone digitizing system 25, and is part of a bone digitizer 30 that features inertial sensor unit 31. The inertial sensor unit 31 may have any appropriate type of inertial sensor, to provide 3-axis orientation tracking. For instance, the inertial sensor unit 31 may have sets of accelerometers and/or gyroscopes, etc. The inertial sensor unit may be known as a sourceless sensor unit, as a micro-electromechanical sensor unit, etc. As mentioned above, the inertial sensor unit 31 is matingly received in the inertial sensor support 23 in a predetermined complementary connection, such that the initializing of the inertial sensor unit 31 will have the inertial sensor unit 31 specifically oriented relative to the X-Y-Z coordinate system illustrated in Fig. 1 (with the Z axis being the cross-product of the X and Y axes).

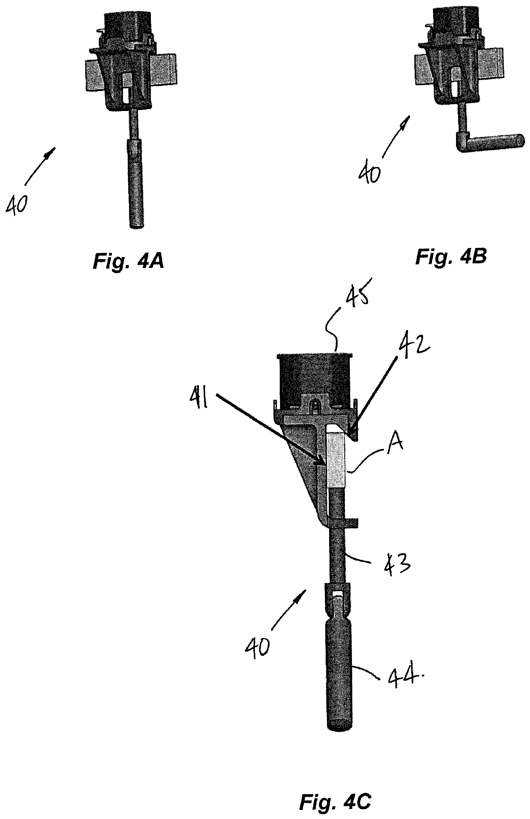

Still referring to Fig. 2, the bone digitizing system 25 may also comprise a table reference 40. Referring to Figs. 4A, 4B and 4C, the table reference 40 is of the type comprising a body for planar engagement with the table plane and a flat surface for planar engagement with a lateral side of the table. In Figs. 4A-4C, the table reference 40 has a body configured to attach to a rail of the table, with a bracket 41 accommodating the rail A in a lateral coplanar connection. A hook-like portion 42 faces the bracket 41 and hooks onto a top edge surface of the rail A. In order to fix the table reference 40 to the rail A, a bolt 43 may be screwingly engaged to a bottom of the bracket 41, with a pivotable handle 44 by which the bolt 43 may be tightened to block the table reference 40 against the rail A, in the manner shown in Figs. 4A-4C, with the bracket 41 having its main surface parallel to that of the rail A. This configuration is one of numerous arrangements the table reference 40 may take.

The table reference 40 may comprise an inertial sensor unit 45 to produce a normal to the table plane and a normal to the table side (resulting in a table lateral axis). Accordingly, the table reference 40 is used to find a plane of support table B upon which the patient lies.

The table reference 40 may be combined with the optional bone digitizer 30, to determine the coordinate system of the pelvis A, in the pelvic application. Accordingly, the bone digitizing system 25 used in a pelvic application produces a pelvic frame of reference 50 for the subsequent navigation of tools relative to the pelvis A. The frame of reference 50 may be attached to a trackable reference (e.g., with 3-axis inertial sensors) in a secured relation relative to the bone.

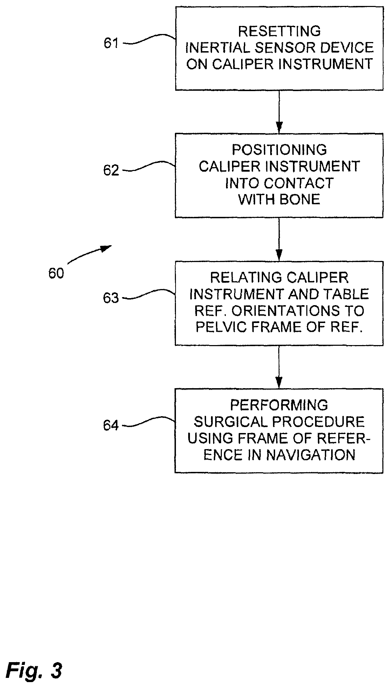

Now that the various components of Figs. 1 and 2 have been described, a method for creating a frame of reference using inertial sensors for subsequent tool navigation is described in further detail with reference to Fig. 3, and is generally shown as 60.

According to 61, the inertial sensor unit 31 is reset once installed in the support caliper instrument 10. According to the embodiment of Figs. 1 and 2, the resetting is facilitated by the complementary connection of the inertial sensor unit 31 in the inertial sensor support 23. According to an embodiment, the calibration is such that the X-Y-Z axes illustrated in Fig. 1 correspond to a 3-axis coordinate system of the inertial sensor unit 31. Accordingly, once the inertial sensor unit 31 is reset, an orientation of the caliper instrument 10 is known, for instance along the longitudinal axis of the caliper instrument 10, shown as the X-axis in Fig. 1.

According to 62, the caliper instrument 10 is positioned into contact with the bone. When the method 60 is used with the pelvis, the length of the caliper instrument in the X direction is set for the pointy ends 14 to be in contact with landmarks of the bone. When the patient is in supine decubitus or lateral decubitus, the landmarks may be the anterior (or posterior) superior iliac spines on both sides of the pelvis. As a result, a mediolateral (ML) axis of the pelvis may be set in the inertial sensor unit 31 when the caliper instrument 10 is in contact with the anterior superior iliac spines, with the legs 12 being arranged to be of the same height (in supine decubitus) or parallel to the table plane normal (in lateral decubitus).

According to 63, it may be desired to relate the table reference 40 to a reference orientation. For instance, the patient in supine decubitus lies on the support table B, and the plane normal of the support table B is used to define an antero-posterior axis of the pelvis, if the patient is in a strict supine decubitus, or quasi-strict supine decubitus. Accordingly, as shown in Fig. 2, the table reference 40 may be used to provide a normal to the table plane. If the patient is aligned with the table B, the ML axis may be in alignment with one of the axes of the table reference 40, for the normal to the table plane to be transferred between the table reference 40 and the bone digitizer 30. If the patient lies in lateral decubitus on the support table B and is aligned with table edges, the lateral axis of the support table B is used to define the AP axis of the pelvis. Accordingly, as shown in Fig. 2, the table reference 40 may be used to provide the lateral axis of the support table B. By relating the table reference 40 to the reference orientation as set forth in 63, the inertial sensor units of the table reference 40 and that of the pelvic frame of reference 50 communicate information so as to transfer the normal of the plane table (supine decubitus) or the lateral axis of the table support (lateral decubitus) to the pelvic frame of reference 50, thereby defining the AP axis of the patient. As also set forth in 63, the inertial sensor units of the caliper instrument 10 and that of the pelvic frame of reference 50 communicate information so as to transfer the X axis of the caliper instrument to the pelvic frame of reference 50, thereby defining the ML axis of the patient. A cross-product of the ML axis and of the AP axis is the longitudinal axis of the patient.

In lateral decubitus, a reference orientation can also be defined such that the table plane normal provides the patient ML axis and the table lateral axis provides the patient antero-posterior axis. In supine decubitus, a reference orientation can also be defined such that the table plane normal provides the patient antero-posterior axis and the table lateral axis provides the patient medio-lateral axis. By relating the table reference 40 to the reference orientation as set forth in 63, the inertial sensor units of the table reference 40 and that of the pelvic frame of reference 50 communicate information so as to transfer the table normal and lateral axis to the pelvic frame of reference 50, thereby defining a ML axis and an antero-posterior axis of the patient. A cross-product of the medio-lateral axis and of the antero-posterior axis is the longitudinal axis of the patient.

According to 63, the inertial sensor units communicate their relative position by rotating the support table around its lateral axis (Trendelenburg/reverse Trendelenburg), using the algorithm described in PCT international publication no. WO 2011/088541 with the table being the object of the calibration, where the two sensor units are fixed relative to each other. If using the caliper instrument 10, the sensor unit on the caliper instrument 10 can rotate around the axis between the legs 12 since only the orientation of that axis, compared to the other inertial sensor unit, is used. The algorithm used to compute the relative position between two inertial sensors device would need to be adapted to compensate for that motion.

According to 64, the surgical procedure may be performed using the frame of reference that has been defined in the previous step for bone navigation, and transferred to any appropriate pelvic reference.

## Claims

1. A digitizer device (10) comprising:
an elongated body (20);
legs (12) connected to the elongated body, wherein each leg has a single free end (14) that is pointy shaped, and wherein the free ends (14) of the legs face each other;
at least one joint between the legs and the elongated body such that free ends of the legs are displaceable relative to one another; and
an inertial sensor unit (31) connected to the elongated body, the inertial sensor unit having a preset orientation aligned with the elongated body.

2. The digitizer device (10) according to claim 1, wherein the at least one joint comprises a translational joint (21) in the elongated body (20).

3. The digitizer device (10) according to claim 2, wherein the translational joint (21) is a telescopic joint between members of the elongated body.

4. The digitizer device (10) according to claim 2, further comprising a locking device (22) on the translational joint (21) to manually lock the joint.

5. The digitizer device (10) according to any one of claims 1 to 4, further comprising a receptacle in the elongated body for releasably receiving the inertial sensor unit in such a way that the preset orientation of the inertial sensor unit is aligned with the elongated body.

6. The digitizer device (10) according to any one of claims 1 to 5, wherein the at least one joint comprises translational joints (13) on each said leg, to adjust a distance between the free ends (14) and the elongated body (20).

7. The digitizer device (10) according to claim 6, wherein the translational joints (13) on each said leg are indexing joints.

8. The digitizer device (10) according to any one of claims 1 to 7, wherein the preset orientation of the inertial sensor unit has an axis parallel to the legs.

9. The digitizer device (10) according to any one of claims 1 to 8, wherein the preset orientation of the inertial sensor unit (31) has an axis parallel to the elongated body (20).

10. An assembly of a digitizer device (10) and table reference device (40) comprising:
the digitizer device being according to any one of claims 1 to 9;
the table reference device comprising:
a body adapted to be fixed to an operating table; and
an inertial sensor unit (45) with a preset orientation related to the operating table;
a patient coordinate system comprising orientation data obtained from the inertial sensor units of the digitizer device and the table reference device.

11. The assembly according to claim 10, further comprising a receptacle in the body of the table reference device (40) for releasably receiving the inertial sensor unit (45) in such a way that the preset orientation of the inertial sensor unit of the table reference device is aligned with a plane of the receptacle.

12. The assembly according to any one of claims 10 and 11, wherein the body of the table reference device comprises a bracket (41) and hook (42) for attachment to a rail of the operating table.

13. The assembly according to any one of claims 10 to 12, wherein the preset orientation of the inertial sensor unit (45) in the table reference device has an axis normal to plane of the table.

14. A method for creating at least part of a pelvic coordinate system of a patient in supine decubitus, comprising:
adjusting a length between ends of a digitizer device (10) to a distance between opposite landmarks of a pelvis of the patient, wherein the digitizer device is according to any one of claims 1 to 9;
applying the ends of the digitizer device against the opposite landmarks of the pelvis; and
initializing an inertial sensor unit (31) of the digitizer device to set an orientation of the digitizer device relative to a medio-lateral axis of the patient,
whereby the medio-lateral axis of the patient is part of the pelvic coordinate system.

15. The method according to claim 14, further comprising positioning a table reference device (40) on an operating table supporting the patient in supine decubitus, and initializing an inertial sensor unit (45) of the table reference device to set an orientation of the table reference device relative to a support plane of the table.

## Patentansprüche

1. Digitalisiervorrichtung (10), umfassend:
einen länglichen Körper (20);
Beine (12), die mit dem länglichen Körper verbunden sind, wobei jedes Bein ein einzelnes freies Ende (14) aufweist, das spitz geformt ist, und wobei die freien Enden (14) der Beine einander zugewandt sind;
mindestens ein Gelenk zwischen den Beinen und dem länglichen Körper, sodass freie Enden der Beine relativ zueinander verschiebbar sind; und
eine Trägheitssensoreinheit (31), die mit dem länglichen Körper verbunden ist, wobei die Trägheitssensoreinheit eine vorgegebene Orientierung aufweist, die nach dem länglichen Körper ausgerichtet ist.

2. Digitalisiervorrichtung (10) gemäß Anspruch 1, wobei das mindestens eine Gelenk ein Translationsgelenk (21) im länglichen Körper (20) umfasst.

3. Digitalisiervorrichtung (10) gemäß Anspruch 2, wobei das Translationsgelenk (21) ein Teleskopgelenk zwischen Gliedern des länglichen Körpers ist.

4. Digitalisiervorrichtung (10) gemäß Anspruch 2, ferner umfassend eine Verriegelungsvorrichtung (22) auf dem Translationsgelenk (21), um das Gelenk manuell zu verriegeln.

5. Digitalisiervorrichtung (10) gemäß einem der Ansprüche 1 bis 4, ferner umfassend eine Aufnahme im länglichen Körper zum lösbaren Aufnehmen der Trägheitssensoreinheit auf eine derartige Weise, dass die vorgegebene Richtung der Trägheitssensoreinheit nach dem länglichen Körper ausgerichtet ist.

6. Digitalisiervorrichtung (10) gemäß einem der Ansprüche 1 bis 5, wobei mindestens ein Gelenk Translationsgelenke (13) auf jedem Bein umfasst, um einen Abstand zwischen den freien Enden (14) und dem länglichen Körper (20) einzustellen.

7. Digitalisiervorrichtung (10) gemäß Anspruch 6, wobei die Translationsgelenke (13) auf jedem Bein Schaltgelenke sind.

8. Digitalisiervorrichtung (10) gemäß einem der Ansprüche 1 bis 7, wobei die vorgegebene Orientierung der Trägheitssensoreinheit eine Achse aufweist, die zu den Beinen parallel liegt.

9. Digitalisiervorrichtung (10) gemäß einem der Ansprüche 1 bis 8, wobei die vorgegebene Orientierung der Trägheitssensoreinheit (31) eine Achse aufweist, die zum länglichen Körper (20) parallel ist.

10. Eine Baugruppe aus einer Digitalisiervorrichtung (10) und Tischreferenzvorrichtung (40), umfassend:
die Digitalisiervorrichtung gemäß einem der Ansprüche 1 bis 9;
die Tischreferenzvorrichtung, umfassend:
einen Körper, der angepasst ist, um an einem Operationstisch fixiert zu werden; und
eine Trägheitssensoreinheit (45) mit einer vorgegebenen Orientierung, die auf den Operationstisch bezogen ist;
ein Patientenkoordinatensystem, umfassend Orientierungsdaten, die von den Trägheitssensoreinheiten der Digitalisiervorrichtung und der Tischreferenzvorrichtung erhalten werden.

11. Baugruppe gemäß Anspruch 10, ferner umfassend eine Aufnahme im Körper der Tischreferenzvorrichtung (40) zum lösbaren Aufnehmen der Trägheitssensoreinheit (45) auf eine derartige Weise, dass die vorgegebene Orientierung der Trägheitssensoreinheit der Tischreferenzvorrichtung nach einer Ebene der Aufnahme ausgerichtet ist.

12. Baugruppe gemäß einem der Ansprüche 10 und 11, wobei der Körper der Tischreferenzvorrichtung eine Halterung (41) und einen Haken (42) zum Befestigen an einer Schiene des Operationstisches umfasst.

13. Baugruppe gemäß einem der Ansprüche 10 bis 12, wobei die vorgegebene Orientierung der Trägheitssensoreinheit (45) in der Tischreferenzvorrichtung eine Achse aufweist, die zur Ebene des Tisches senkrecht ist.

14. Verfahren zum Erzeugen von mindestens einem Teil eines Beckenkoordinatensystems für einen Patienten in Supination decubitus, umfassend:
Einstellen einer Länge zwischen Enden einer Digitalisiervorrichtung (10) auf einen Abstand zwischen entgegengesetzten Referenzpunkten eines Beckens des Patienten, wobei die Digitalisiervorrichtung gemäß einem der Ansprüche 1 bis 9 ist;
Anwenden der Enden der Digitalisiervorrichtung gegen die entgegengesetzten Referenzpunkte des Beckens, und
Initialisieren einer Trägheitssensoreinheit (31) der Digitalisiervorrichtung, um eine Orientierung der Digitalisiervorrichtung relativ zu einer medio-lateralen Achse des Patienten festzulegen,
wobei die medio-laterale Achse des Patienten ein Teil des Beckenkoordinatensystems ist.

15. Verfahren gemäß Anspruch 14, das ferner das Positionieren einer Tischreferenzvorrichtung (40) auf einem Operationstisch, der den Patienten in Supination decubitus stützt, und Initialisieren einer Trägheitssensoreinheit (45) der Tischreferenzvorrichtung, um eine Orientierung der Tischreferenzvorrichtung relativ zu einer Stützebene des Tisches festzulegen, umfasst.

## Revendications

1. Dispositif numériseur (10), comprenant :
un corps allongé (20) ;
des branches (12) reliées au corps allongé, chaque branche ayant une extrémité libre (14) unique de forme pointue et les extrémités libres (14) des branches se faisant face ;
au moins une articulation entre les branches et le corps allongé de telle façon que les extrémités libres des branches peuvent se déplacer l'une par rapport à l'autre ; et
un ensemble capteur inertiel (31) relié au corps allongé, l'ensemble capteur inertiel ayant une orientation prédéterminée alignée avec le corps allongé.

2. Dispositif numériseur (10) selon la revendication 1, dans lequel ladite au moins une articulation comprend une articulation de translation (21) dans le corps allongé (20) .

3. Dispositif numériseur (10) selon la revendication 2, dans lequel l'articulation de translation (21) est une articulation télescopique entre des éléments du corps allongé.

4. Dispositif numériseur (10) selon la revendication 2, comprenant en outre un dispositif de verrouillage (22) sur l'articulation de translation (21) afin de verrouiller manuellement l'articulation.

5. Dispositif numériseur (10) selon l'une quelconque des revendications 1 à 4, comprenant en outre un réceptacle dans le corps allongé afin de recevoir l'ensemble capteur inertiel de manière libérable de telle façon que l'orientation prédéterminée de l'ensemble capteur inertiel est alignée avec le corps allongé.

6. Dispositif numériseur (10) selon l'une quelconque des revendications 1 à 5, dans lequel ladite au moins une articulation comprend des articulations de translation (13) sur chacune desdites branches afin d'ajuster une distance entre les extrémités libres (14) et le corps allongé (20).

7. Dispositif numériseur (10) selon la revendication 6, dans lequel les articulations de translation (13) sur chacune desdites branches sont des articulations à indexage.

8. Dispositif numériseur (10) selon l'une quelconque des revendications 1 à 7, dans lequel l'orientation prédéterminée de l'ensemble capteur inertiel a un axe parallèle aux branches.

9. Dispositif numériseur (10) selon l'une quelconque des revendications 1 à 8, dans lequel l'orientation prédéterminée de l'ensemble capteur inertiel (31) a un axe parallèle au corps allongé (20) .

10. Assemblage d'un dispositif numériseur (10) et d'un dispositif de référence de table (40), comprenant :
le dispositif numériseur selon l'une quelconque des revendications 1 à 9 ;
le dispositif de référence de table comprenant :
un corps adapté pour être fixé sur une table d'opération ; et
un ensemble capteur inertiel (45) avec une orientation prédéterminée par rapport à la table d'opération ;
un système de coordonnées de patient comprenant des données d'orientation obtenues à partir des ensembles capteurs inertiels du dispositif numériseur et du dispositif de référence de table.

11. Assemblage selon la revendication 10, comprenant en outre un réceptacle dans le corps du dispositif de référence de table (40) afin de recevoir de manière libérable l'ensemble capteur inertiel (45) de telle façon que l'orientation prédéterminée de l'ensemble capteur inertiel du dispositif de référence de table est alignée avec un plan du réceptacle.

12. Assemblage selon l'une quelconque des revendications 10 et 11, dans lequel le corps du dispositif de référence de table comprend un support (41) et un crochet (42) pour la fixation à un rail de la table d'opération.

13. Assemblage selon l'une quelconque des revendications 10 à 12, dans lequel l'orientation prédéterminée de l'ensemble capteur inertiel (45) dans le dispositif de référence de table a un axe perpendiculaire au plan de la table.

14. Procédé de création d'au moins une partie d'un système de coordonnées pelviennes d'un patient en décubitus dorsal, comprenant :
l'ajustement d'une longueur entre des extrémités d'un dispositif numériseur (10) à une distance entre des repères opposés du bassin du patient, le dispositif numériseur étant selon l'une quelconque des revendications 1 à 9 ;
l'application des extrémités du dispositif numériseur contre les repères opposés du bassin ; et
l'initialisation d'un ensemble capteur inertiel (31) du dispositif numériseur pour définir une orientation du dispositif numériseur par rapport à un axe médio-latéral du patient,
moyennant quoi l'axe médio-latéral du patient fait partie du système de coordonnées pelviennes.

15. Procédé selon la revendication 14, comprenant en outre le positionnement d'un dispositif de référence de table (40) sur une table d'opération supportant le patient en décubitus dorsal, et l'initialisation d'un ensemble capteur inertiel (45) du dispositif de référence de table pour définir une orientation du dispositif de référence de table par rapport à un plan de support de la table.
